# EUROPEAN PATENT SPECIFICATION

(11) **EP 4 442 804 B1**
(45) Date of publication and mention of the grant of the patent: **11.06.2025**
(21) Application number: 24156831.0
(22) Date of filing: 09.02.2024
(51) Int. Cl.: C12M 1/34, C12M 1/36

(54) **SYSTEMS AND METHODS FOR OPTIMIZING A BIOREACTOR FOR CONTROLLING GROWTH OF HUMAN STEM CELLS**
SYSTEME UND VERFAHREN ZUR OPTIMIERUNG EINES BIOREAKTORS ZUR STEUERUNG DES WACHSTUMS VON MENSCHLICHEN STAMMZELLEN
SYSTÈMES ET PROCÉDÉS D'OPTIMISATION D'UN BIORÉACTEUR POUR RÉGULER LA CROISSANCE DE CELLULES SOUCHES HUMAINES

(30) Priority: 17.03.2023 IN 202321018299
(43) Date of publication of application: 09.10.2024
(73) Proprietor: Tata Consultancy Services Limited, Maharashtra (IN)
(72) Inventor: DIWANJI, Srinivas Prakash, 411013 Pune (IN); RUNKANA, Venkataramana, 411013 Pune (IN); PREMRAJ, Karundev, 560009 Bangalore (IN); BUDDHIRAJU, Venkata Sudheendra, 411013 Pune (IN)
(74) Representative: Goddar, Heinz J.

(56) References cited:
- WO-A1-2019/100040
- US-A1- 2021 230 532
- US-A1- 2022 282 202
- KINNEY MELISSA A. ET AL: "The Multiparametric Effects of Hydrodynamic Environments on Stem Cell Culture", TISSUE ENGINEERING PART B, vol. 17, no. 4, 1 August 2011 (2011-08-01), US, pages 249 - 262, XP093190845, ISSN: 1937-3368, DOI: 10.1089/ten.teb.2011.0040
- SAEED ABBASALIZADEH: "Bioprocess Development for Mass Production of Size-Controlled Human Pluripotent Stem Cell Aggregates in Stirred Suspension Bioreactor", TISSUE ENGINEERING. PART C, METHODS DEC 2008, vol. 18, no. 11, 1 November 2012 (2012-11-01), US, pages 831 - 851, XP093166759, ISSN: 1937-3384, DOI: 10.1089/ten.tec.2012.0161

## Description

### CROSS-REFERENCE TO RELATED APPLICATIONS AND PRIORITY

The present application claims priority to Indian application no. 202321018299, filed on March 17, 2023.

### TECHNICAL FIELD

The disclosure herein generally relates to bioreactor systems, and, more particularly, to systems and methods for optimizing a bioreactor for controlling growth of human stem cells.

### BACKGROUND

Conventionally control and optimization of bioreactors has been challenging due to the unpredictable behaviour of living cells and non-linear process dynamics. Another problem is the unavailability of sensors to measure process parameters and the delay in arrival of offline measurement values from the quality lab which makes controlling more rigid. Therefore, significant challenges are inherently observed with respect to strict Current Good Manufacturing Practice (CGMP) practices, quality standards and ethical aspects of the subject. Although small-scale experiments with murine stem cells have significantly helped in understanding the mechanism of stem cell growth, they cannot be used as a substitute for human stem cells due to differences in sensitivity of cells and doubling rate. Current existing technologies are unable to produce large numbers of pluripotent stem cells required for stem cell therapy. This is due to two main reasons, which are limited knowledge on stem cells growth physics and bioreactor technology for culturing these cells. WO2019100040 describes a computer implemented method to control a cell culture, comprising sensors to measure a plurality of parameters, such as pH, nutrients concentration, temperature, stirring rate, as well as imaging means to monitor size of cell agglomerates. The method uses a model to compare measured results and data stored from similar methods.

### SUMMARY

Embodiments of the present disclosure present technological improvements as solutions to one or more of the above-mentioned technical problems recognized by the inventors in conventional systems.

For example, in one aspect, there is provided a processor implemented method for optimizing a bioreactor for controlling growth of human stem cells. The method comprises receiving, via one or more hardware processors of a bioreactor, a cell culture medium of one or more human stem cells and a sparger supply; obtaining, via the one or more hardware processors, by using a plurality of soft sensors placed at one or more regions of the bioreactor, a soft sensor data pertaining to a first set of parameters and a second set of parameters from the cell culture medium, wherein the first set of parameters comprises an inoculum density, a cell aggregate size, a pH value, a nutrient concentration, a temperature, a by-product concentration, an agitation rate, a sparging rate of the sparger supply, a macro-sparger flow rate, and a feed rate, wherein the second set of parameters comprises a base-addition controller, and wherein the second set of parameters further comprises at least a subset of the first set of parameters; pre-processing, via the one or more hardware processors, the soft sensor data to obtain a plurality of pre-processed sensor values; performing simulation, via the one or more hardware processors, by using a physics based mathematical model, on the plurality of pre-processed sensor values pertaining to the first set of parameters, and a third set of parameters to obtain a set of simulated variables, wherein the third set of parameters are obtained from a domain knowledge database; iteratively optimizing the second set of parameters using the set of simulated variables to obtain a set of optimized real-time operating variables, until a value of a cell density reaches a corresponding predefined threshold, wherein the set of optimized real-time operating variables are obtained to evaluate the cell density using one or more associated constraint values; controlling, via the one or more hardware processors, a variation in a pH value, and a cell aggregate size using one or more control strategies based on the set of simulated variables for the growth of the one or more human stem cells; performing a comparison of the set of optimized real-time operating variables and the second set of parameters to obtain a set of correction factors; sending, via the one or more hardware processors, a plurality of recommended parameter values to a plant automation system based on the set of correction factors; and updating, via the one or more hardware processors, the second set of parameters based on the plurality of recommended parameter values.

In an embodiment, the bioreactor is configured to inspect one or more key parameters pertaining to growth of the one or more human stem cells, and wherein the one or more key parameters comprises the cell density, the cell aggregate size, a nutrient concentration, a by-product concentration, and a dead cell density.

In an embodiment, the step of pre-processing comprises averaging the soft sensor data obtained from the plurality of soft sensors, and wherein the soft sensor data is pre-processed to refrain bias in growth of the one or more human stem cells.

In an embodiment, variation in the pH value ranges from a first pre-defined value to a second pre-defined value.

In an embodiment, the set of simulated variables comprises a change in the cell density, a change in the cell aggregate size, a rate of change in the nutrient consumption, the variation in the pH value, a rate of change in the by-product production, one or more oxygen levels, one or more carbon dioxide levels, an effect of temperature in the bioreactor.

In another aspect, there is provided a processor implemented system for optimizing a bioreactor for controlling growth of human stem cells. The system comprises: a memory storing instructions; one or more communication interfaces; and one or more hardware processors coupled to the memory via the one or more communication interfaces, wherein the one or more hardware processors are configured by the instructions to: receive a cell culture medium of one or more human stem cells and a sparger supply; obtain, by using a plurality of soft sensors placed at one or more regions of the bioreactor, a soft sensor data pertaining to a first set of parameters and a second set of parameters from the cell culture medium, wherein the first set of parameters comprises an inoculum density, a cell aggregate size, a pH value, a nutrient concentration, a temperature, a by-product concentration, an agitation rate, a sparging rate of the sparger supply, a macro-sparger flow rate, and a feed rate, wherein the second set of parameters comprises a base-addition controller, and wherein the second set of parameters further comprises at least a subset of the first set of parameters; pre-process the soft sensor data to obtain a plurality of pre-processed sensor values; perform simulation, by using a physics based mathematical model, on the plurality of pre-processed sensor values pertaining to the first set of parameters, and a third set of parameters to obtain a set of simulated variables, wherein the third set of parameters are obtained from a domain knowledge database; iteratively optimize the second set of parameters using the set of simulated variables to obtain a set of optimized real-time operating variables, until a value of a cell density reaches a corresponding predefined threshold, wherein the set of optimized real-time operating variables are obtained to evaluate the cell density using one or more associated constraint values; control a variation in a pH value, and a cell aggregate size using one or more control strategies based on the set of simulated variables for the growth of the one or more human stem cells; perform a comparison of the set of optimized real-time operating variables and the second set of parameters to obtain a set of correction factors; send a plurality of recommended parameter values to a plant automation system based on the set of correction factors; and update the second set of parameters based on the plurality of recommended parameter values.

In an embodiment, the bioreactor is configured to inspect one or more key parameters pertaining to growth of the one or more human stem cells, and wherein the one or more key parameters comprises the cell density, the cell aggregate size, a nutrient concentration, a by-product concentration, and a dead cell density.

In an embodiment, the step of pre-processing comprises averaging the soft sensor data obtained from the plurality of soft sensors, and wherein the soft sensor data is pre-processed to refrain bias in growth of the one or more human stem cells.

In an embodiment, variation in the pH value ranges from a first pre-defined value to a second pre-defined value.

In an embodiment, the set of simulated variables comprises a change in the cell density, a change in the cell aggregate size, a rate of change in the nutrient consumption, the variation in the pH value, a rate of change in the by-product production, one or more oxygen levels, one or more carbon dioxide levels, an effect of temperature in the bioreactor.

In yet another aspect, there are provided one or more non-transitory machine-readable information storage mediums comprising one or more instructions which when executed by one or more hardware processors cause optimizing a bioreactor for controlling growth of human stem cells by receiving a cell culture medium of one or more human stem cells and a sparger supply; obtaining, by using a plurality of soft sensors placed at one or more regions of the bioreactor, a soft sensor data pertaining to a first set of parameters and a second set of parameters from the cell culture medium, wherein the first set of parameters comprises an inoculum density, a cell aggregate size, a pH value, a nutrient concentration, a temperature, a by-product concentration, an agitation rate, a sparging rate of the sparger supply, a macro-sparger flow rate, and a feed rate, wherein the second set of parameters comprises a base-addition controller, and wherein the second set of parameters further comprises at least a subset of the first set of parameters; pre-processing the soft sensor data to obtain a plurality of pre-processed sensor values; performing simulation, by using a physics based mathematical model, on the plurality of pre-processed sensor values pertaining to the first set of parameters, and a third set of parameters to obtain a set of simulated variables, wherein the third set of parameters are obtained from a domain knowledge database; iteratively optimizing the second set of parameters using the set of simulated variables to obtain a set of optimized real-time operating variables, until a value of a cell density reaches a corresponding predefined threshold, wherein the set of optimized real-time operating variables are obtained to evaluate the cell density using one or more associated constraint values; controlling a variation in a pH value, and a cell aggregate size using one or more control strategies based on the set of simulated variables for the growth of the one or more human stem cells; performing a comparison of the set of optimized real-time operating variables and the second set of parameters to obtain a set of correction factors; sending a plurality of recommended parameter values to a plant automation system based on the set of correction factors; and updating the second set of parameters based on the plurality of recommended parameter values.

In an embodiment, the bioreactor is configured to inspect one or more key parameters pertaining to growth of the one or more human stem cells, and wherein the one or more key parameters comprises the cell density, the cell aggregate size, a nutrient concentration, a by-product concentration, and a dead cell density.

In an embodiment, the step of pre-processing comprises averaging the soft sensor data obtained from the plurality of soft sensors, and wherein the soft sensor data is pre-processed to refrain bias in growth of the one or more human stem cells.

In an embodiment, variation in the pH value ranges from a first pre-defined value to a second pre-defined value.

In an embodiment, the set of simulated variables comprises a change in the cell density, a change in the cell aggregate size, a rate of change in the nutrient consumption, the variation in the pH value, a rate of change in the by-product production, one or more oxygen levels, one or more carbon dioxide levels, an effect of temperature in the bioreactor.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 depicts an exemplary system for optimizing a bioreactor for controlling growth of human stem cells, in accordance with an embodiment of the present disclosure.
FIG. 2 depicts an exemplary high level block diagram of the system of FIG. 1 for optimizing a bioreactor for controlling growth of human stem cells, in accordance with an embodiment of the present disclosure
FIG. 3 depicts an exemplary flow chart illustrating a method for optimizing a bioreactor for controlling growth of human stem cells, using the systems of FIGS. 1-2, in accordance with an embodiment of the present disclosure.
FIG. 4 depicts an exemplary block diagram of a model library as implemented by the systems 100 of FIG. 1 and 2, in accordance with an embodiment of the present disclosure.
FIG. 5 depicts a graphical representation illustrating a cell concentration profile with respect to time for the bioreactor, in accordance with an embodiment of the present disclosure.
FIG. 6A depicts a graphical representation illustrating a cell concentration profile for a non-aggregate disruption (NAD) scenario of the bioreactor, in accordance with an embodiment of the present disclosure.
FIG. 6B depicts a graphical representation illustrating a glucose concentration profile for the non-aggregate disruption (NAD) scenario of the bioreactor, in accordance with an embodiment of the present disclosure.
FIG. 6C depicts a graphical representation illustrating an aggregate cell size for the non-aggregate disruption (NAD) scenario of the bioreactor, in accordance with an embodiment of the present disclosure.
FIG. 6D depicts a graphical representation illustrating a lactic acid concentration profile for the non-aggregate disruption (NAD) scenario of the bioreactor, in accordance with an embodiment of the present disclosure.
FIG. 6E depicts a graphical representation illustrating an oxygen concentration profile for the non-aggregate disruption (NAD) scenario of the bioreactor, in accordance with an embodiment of the present disclosure.
FIG. 7A depicts a graphical representation illustrating the cell concentration profile for the aggregate disruption (AD) scenario of the bioreactor, in accordance with an embodiment of the present disclosure.
FIG. 7B depicts a graphical representation illustrating the glucose concentration profile for the aggregate disruption (AD) scenario of the bioreactor, in accordance with an embodiment of the present disclosure.
FIG. 7C depicts a graphical representation illustrating the aggregate cell size for the aggregate disruption (AD) scenario of the bioreactor, in accordance with an embodiment of the present disclosure.
FIG. 7D depicts a graphical representation illustrating the lactic acid concentration profile for the aggregate disruption (AD) scenario of the bioreactor, in accordance with an embodiment of the present disclosure.
FIG. 7E depicts a graphical representation illustrating an oxygen concentration profile for the aggregate disruption (AD) scenario of the bioreactor, in accordance with an embodiment of the present disclosure.

### DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Conventionally control and optimization of bioreactor has been challenging due to the unpredictable behaviour of living cells and non-linear process dynamics. Another problem is unavailability of sensors to measure process parameters and delay in arrival of offline measurement values from the quality lab which makes controlling more rigid. Therefore, it inherently comes with significant challenges with respect to strict Current Good Manufacturing Practice (CGMP) practices, quality standards and ethical aspect of the subject. Although small-scale experiments with murine stem cells have significantly helped in understanding the mechanism of stem cell growth, they cannot be used as a substitute for human stem cells due to differences in sensitivity of cells and doubling rate. Current existing technologies are unable to produce large numbers of pluripotent stem cells required for stem cell therapy. This is due to two main reasons which are limited knowledge on stem cells growth physics and bioreactor technology for culturing these cells.

Present disclosure provides systems and methods that implement a mathematical model (e.g., physics-based models) to simulate the expansion/growth of human stem cells in a given cell culture media condition. An optimization module is implemented which provides with the appropriate values of the adjustable parameters to optimize the quantity and quality yield of human stem cells. The mathematical model (physics-based model) evaluates the total cell density, viable cell density, and number of dead cells. Further it evaluates the cell aggregate size formation and also can be used to study of effect of inoculum variation before implementing a batch setup. The model further provides information about the rate of consumption of nutrients such as glucose, glutamine, oxygen in batch as well as fed-batch reactors which also enables in optimizing the feed rate, sparging rate etc. The effect of nutrient concentration variation, sparging rate is analysed from here. By-product formation through the cell metabolism processes is also captured through which concentrations of lactic acid, and carbon dioxide is evaluated. The effect of pH on growth and quality of stem cells is well known but has not been yet quantified. Here the mathematical model also evaluates the variation of pH during the bioreactor operation and its effect on the human stem cell growth. Further the mathematical model also captures the effect of temperature on all of the cell culture medium. Furthermore, optimized parameter values are provided by the system for feed rate, sparging rate, fed-batch frequency, etc. for obtaining higher yield of human stem cells. The system also includes optimizing the bioreactor to ensure uniform hydrodynamics condition for uniformity in the product yield.

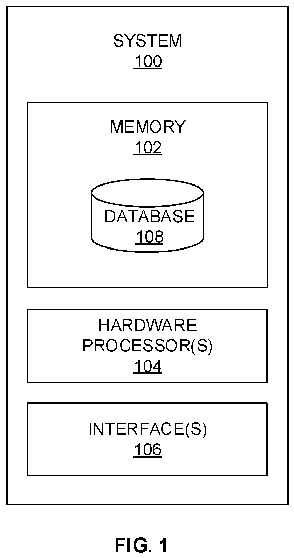

Referring now to the drawings, and more particularly to FIGS. 1 through 7E, where similar reference characters denote corresponding features consistently throughout the figures, there are shown preferred embodiments, and these embodiments are described in the context of the following exemplary system and/or method.

FIG. 1 depicts an exemplary system 100 for optimizing a bioreactor for controlling growth of human stem cells, in accordance with an embodiment of the present disclosure. In an embodiment, the system 100 includes one or more hardware processors 104, communication interface device(s) or input/output (I/O) interface(s) 106 (also referred as interface(s)), and one or more data storage devices or memory 102 operatively coupled to the one or more hardware processors 104. The one or more processors 104 may be one or more software processing components and/or hardware processors. In an embodiment, the hardware processors can be implemented as one or more microprocessors, microcomputers, microcontrollers, digital signal processors, central processing units, state machines, logic circuitries, and/or any devices that manipulate signals based on operational instructions. Among other capabilities, the processor(s) is/are configured to fetch and execute computer-readable instructions stored in the memory. In an embodiment, the system 100 can be implemented in a variety of computing systems, such as laptop computers, notebooks, hand-held devices (e.g., smartphones, tablet phones, mobile communication devices, and the like), workstations, mainframe computers, servers, a network cloud, and the like.

The I/O interface device(s) 106 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the I/O interface device(s) can include one or more ports for connecting a number of devices to one another or to another server.

The memory 102 may include any computer-readable medium known in the art including, for example, volatile memory, such as static random-access memory (SRAM) and dynamic-random access memory (DRAM), and/or non-volatile memory, such as read only memory (ROM), erasable programmable ROM, flash memories, hard disks, optical disks, and magnetic tapes. In an embodiment, a database 108 is comprised in the memory 102, wherein the database 108 comprises information pertaining to a cell culture medium of one or more human stem cells and a sparger supply, a soft sensor data pertaining to a first set of parameters and a second set of parameters from the cell culture medium, a plurality of pre-processed sensor values The database 108 further comprises a set of optimized real-time operating variables, controlled pH values, controlled cell aggregate sizes, a set of correction factors, a plurality of recommended parameter values for obtaining optimized bioreactor and controlled growth of human stem cells, and the like. The memory 102 comprises one or more models such as but are not limited to, a physics based mathematical model, and one or more control strategies, and the like. The memory 102 further comprises (or may further comprise) information pertaining to input(s)/output(s) of each step performed by the systems and methods of the present disclosure. In other words, input(s) fed at each step and output(s) generated at each step are comprised in the memory 102 and can be utilized in further processing and analysis.

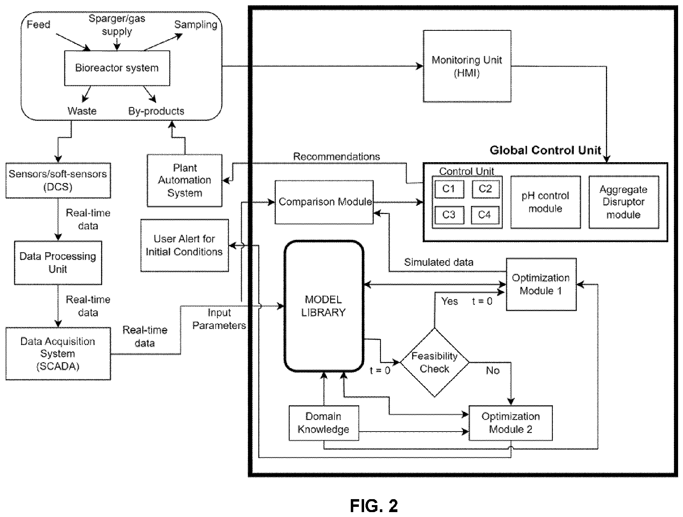

FIG. 2, with reference to FIG. 1, depicts an exemplary high level block diagram of the system 100 for optimizing a bioreactor for controlling growth of human stem cells, in accordance with an embodiment of the present disclosure.

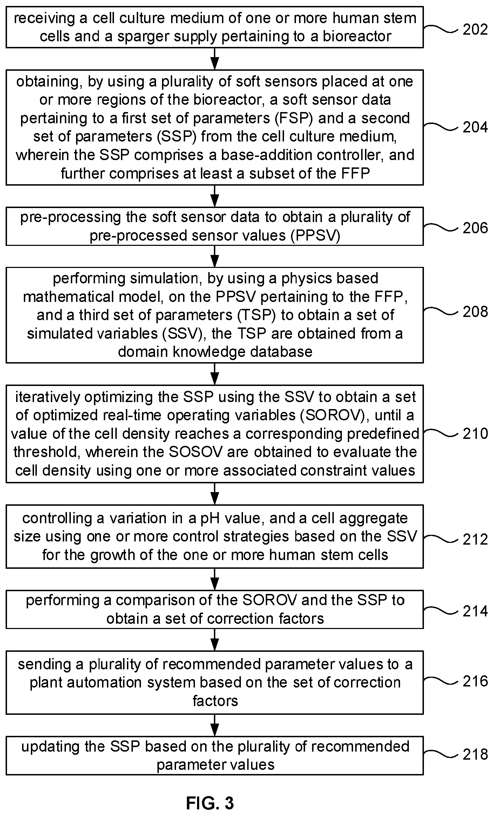

FIG. 3 depicts an exemplary flow chart illustrating a method for optimizing a bioreactor for controlling growth of human stem cells, using the systems 100 of FIG. 1-2, in accordance with an embodiment of the present disclosure. In an embodiment, the system(s) 100 comprises one or more data storage devices or the memory 102 operatively coupled to the one or more hardware processors 104 and is configured to store instructions for execution of steps of the method by the one or more processors 104. The steps of the method of the present disclosure will now be explained with reference to components of the system 100 of FIG. 1, the block diagram of the system 100 depicted in FIG. 2, and the flow diagram as depicted in FIG. 3.

At step 202 of the method of the present disclosure, the one or more hardware processors 104 receive a cell culture medium of one or more human stem cells and a sparger supply. Bioreactor forms a part of the biopharma industry, and is a vessel that supports cell growth, in the cell culture medium. Depending on the operation pertaining to setup of the system 100, the bioreactor can be a batch reactor, a fed-batch reactor, or a continuous fed-batch reactor. In case of the fed-batch reactor and the continuous fed-batch reactor, a certain feed of cell culture medium is provided into the bioreactor in some intervals of time. This interval of time is considered by a mathematical model to simulate human stem cells growth. Sparger/gas supply is the air that is sparged into vessel of the bioreactor. The sparging can be continuous or intermittent which is considered in the mathematical model. Oxygen as a nutrient is provided by sparging. In an embodiment of the present disclosure, macro-spargers are used to drive out excess carbon dioxide (CO2) from the cell culture medium. This is done by sparging Nitrogen into the culture medium as nitrogen bubbles are difficult to dissolve in the cell culture medium and they drive out excess CO2 with them to the headspace above the bioreactor culture volume. Cell culturing process takes many days to complete (e.g., 4-10 days) depending on the bioreactor volume and the yield quantity required. The bioreactor is equipped with sampling provision where a sample can be collected after some period of time in order to inspect the cell growth attributes. This includes cell density, aggregate size, nutrient concentration, by-product concentration, dead cell density, and the like. Waste and by-products are produced as a result of the cell metabolism process and the one or more control strategies are employed in the system accordingly. By-products include lactic acid formation, carbon dioxide (CO2) release into the cell culture medium. In pH control strategy, base addition is done to control the pH value. Excess base forms waste and dead cells in the cell culture medium.

The bioreactor is configured to inspect one or more key parameters pertaining to growth of the one or more human stem cells. The one or more key parameters comprises the cell density, the cell aggregate size, a nutrient concentration, a by-product concentration, and a dead cell density, in one embodiment of the present disclosure.

Referring to steps of FIG. 3, at step 204 of the method of the present disclosure, the one or more hardware processors 104 obtain by using a plurality of soft sensors placed at one or more regions of the bioreactor, a soft sensor data pertaining to a first set of parameters and a second set of parameters from the cell culture medium. Distributed Control System (DCS) is implemented by the method and system of the present disclosure (e.g., refer FIG. 2) to have one or more sensors placed at critical regions of bioreactors for collecting soft sensor data from the cell culture medium. In other words, the setup of the bioreactor is equipped with a variety of sensors/soft sensors to collect information on critical parameters of the cell culture medium. The first set of parameters may also be referred as input parameters and are interchangeably used herein. The first set of parameters comprises an inoculum density, a cell aggregate size, a pH value, a nutrient concentration, a temperature, a by-product concentration, an agitation rate, a sparging rate of the sparger supply, a macro-sparger flow rate, and a feed rate. The second set of parameters may also be referred as one or more real-time operating parameters and are interchangeably used herein. The second set of parameters comprises a base-addition controller, and the like. The second set of parameters further comprises at least a subset of the first set of parameters. For instance, the second set of parameters include the temperature, the agitation rate, the sparger flow rate, the macro-sparger flow rate, and the feed rate (in case of fed-batch and continuous fed-batch bioreactors) which are part of the first set of parameters.

At step 206 of the method of the present disclosure, the one or more hardware processors 104 pre-process the soft sensor data to obtain a plurality of pre-processed sensor values. The real-time data from the soft sensors is then processed in the data pre-processing unit (e.g., refer FIG. 2). The values of critical parameters (e.g., the first set and the second set of parameters) are recorded at various locations of the bioreactor. These values are an indication of human stem cell growth happening at various locations. Averaging process is done on these values. In this way, the simulation of human stem cell growth by the mathematical model does not result in bias in the human stem cell growth at various locations. In other words, the step of pre-processing comprises averaging the soft sensor data obtained from the plurality of soft sensors wherein the soft sensor data is pre-processed to refrain (or prevent/avoid) bias in growth of the one or more human stem cells.

At step 208 of the method of the present disclosure, the one or more hardware processors 104 perform simulation, by using a physics based mathematical model, on the plurality of pre-processed sensor values pertaining to the first set of parameters, and a third set of parameters to obtain a set of simulated variables. The third set of parameters are obtained from a domain knowledge database. The domain knowledge database may be either part of the database 104 or stored in the memory 102. The key parameters from the domain knowledge database are fed into the mathematical model such as pH setpoints, cell aggregate size threshold limits, toxicity due to increase in the by-products concentration. One or more limits for the mathematical model parameters which are physics-dependent are also specified, in an embodiment of the present disclosure. The domain knowledge database is also queried by the one or more hardware processors 104 to send relevant information on human stem cells growth physics for optimization in order to ensure that one or more parameters assume the value which are conducive to the human stem cells growth.

At step 210 of the method of the present disclosure, the one or more hardware processors 104 iteratively optimize the second set of parameters using the set of simulated variables to obtain a set of optimized real-time operating variables, until a value of a cell density reaches a corresponding predefined threshold. The set of optimized real-time operating variables are obtained to evaluate the cell density using one or more associated constraint values, in an embodiment of the present disclosure.

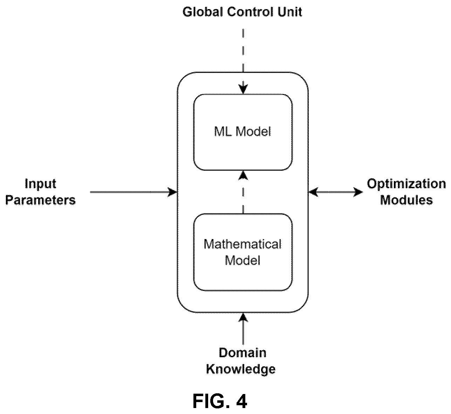

Prior to performing simulation and optimization, the system and method of the present disclosure may perform (or performs) feasibility check on the first set of parameters (e.g., input parameters) and on other input variables which include user alerts with initial conditions. This feasibility check is carried out using a model library via a feasibility module as shown in FIG. 2. The model library includes different kinds of model which can be implemented in a system. One is a physics-based mathematical model (e.g., the mathematical model) and another one is data-based machine learning (ML) model. For the ML model to be used, it is trained and tested on numerous data concerning with large-scale expansion of human stem cells. FIG. 4, with reference to FIGS. 1 through 3, depicts an exemplary block diagram of the model library as implemented by the systems 100 of FIG. 1 and 2, in accordance with an embodiment of the present disclosure. It is recommended to use the physics-based mathematical model when implementing the system and methods for the initial times on a bioreactor system. In the initial times, the ML model is not implemented as it is not yet trained. In such scenarios, it takes additional inputs from the mathematical model and the global control unit which is the training data for the ML model. The ML model consists of a deep neural network (not shown in FIGS.) with one input and one output layer with two or more hidden layers. The input layer takes the inputs from the input parameters (e.g., the first set of parameters) and output variable parameters from the mathematical model. The output labels are from the GCU which consist of operating parameter values and prediction of the growth of human stem cells.

The above description may be better understood by way of the following description. For the case where, the model library is implemented for the first time, it is recommended to use the physics-based mathematical model. The inputs to the model library are the initial conditions and also target viable cell density to be achieved. At the zero-time cycle (initial point before starting the bioreactor cell culture operation), the one or more hardware processors 104 performs a feasibility check to determine whether the target cell density can be achieved for the given initial condition. Initial conditions consist of parameters such as temperature, inoculum density, cell aggregate size, initial nutrient concentration, initial by-product concentration, pH level. Nutrients may consist of glucose, oxygen but are not limited to that. By-products may consist of lactic acid, carbon dioxide but are not limited to that. An example of initial condition can be temperature of 37 degree Celsius, inoculum density of 1*10⁵ cells/ml, cell aggregate size of 50 micrometer, glucose initial concentration of 5.0 g/L, oxygen initial concentration of 0.225 mmol/mm³, by-products concentration of 0 as there are no by-products formed initially at the start of the cycle, and a pH level of 6.95. The mathematical model receives the required input from a data acquisition system (refer FIG. 2), which is the first set of parameters and the second set of parameters. It takes the input as an initial condition for solving coupled ordinary differential equations (ODE) developed and gives desired output. Output from the mathematical model consists of values of variables such as viable cell density, dead cell density, cell aggregate size and its variation, nutrients concentration and its variation, by-products concentration and its variation, pH level and its variation. An example can be after the cell culture process the average cell aggregate size could reach 280-300 micrometer as predicted by the mathematical model.

Feasibility check is a decision-making system. If the target cell density is not achievable, a second optimization module (e.g., an optimization module 2 of FIG. 2) reflects on the initial conditions provided by the bioreactor. The optimization module 2 incorporates appropriate changes into the initial conditions so that the target cell density may be achieved. For example, the inoculum density should be increased or decreased by a certain scale to achieve the required target cell density. These changes are then processed to raise or generate alerts for one or more users (e.g., administrator/operator, and the like) to make changes in the initial conditions. The above implementation of optimization module 2 is better understood by way of following description.

In case of negative feasibility outcome, with the given initial conditions, it is not possible to achieve the target viable cell density. In such a case, the optimization module 2 is triggered. Parameters concerned with initial conditions such as inoculum density, cell aggregate size, initial nutrient concentration, oxygen concentration, bioreactor volume, temperature are optimized in order to achieve the target cell density value. The optimization module 2 receives significant information This significant information is concerned with the upper and lower limits of the initial condition variables which are temperature, inoculum density, cell aggregate size, initial nutrient concentration, initial by-product concentration, pH level. These limits form the constraint values for the optimization problem and some parameters like temperature are dependent on the geographical location on constraint values. Constraint values of the parameters which form the part of initial condition are the upper and lower limit values of the parameters. For example, the temperature can range from 35 degree Celsius to 40 degree Celsius, Inoculum density can range from 0.1*10⁵ cells/ml to 10*10⁵ cells/ml, initial cell aggregate size can range 30 micrometer to 80 micrometer, initial glucose concentration can range from 0.1 g/L to 10.0 g/L, oxygen concentration can range from 0.05 mmol/mm³ to 0.50 mmol/mm³, initial pH level can range from 6.95 to 7.05 pertaining to the parameters being optimized from the domain knowledge database. For example, temperature of the cell culture media cannot go beyond for example 45 degree Celsius. This information is provided by the domain knowledge database which is used in optimization processes.

In case the feasibility check gives a positive outcome, a first optimization module (e.g., an optimization module 1 of FIG. 2) is triggered. The mathematical model solves cell density equation which is related to the growth rate of human stem cells. The growth rate term is in turn dependent on the nutrient concentration, by-product concentration, pH value, etc. The mathematical model further solves aggregate size equation, nutrient consumption equation, pH equation. The mathematical model provides a complete simulation where all the critical parameters in the cell culture medium are taken into consideration and their effects are simulated on the human stem cells growth. Human stem cells are particularly sensitive and require much control of the cell culture media parameters. The mathematical model simulates (e.g., refer simulation step 208) the growth of human stem cells for the whole process duration assuming that the operating parameters and model parameters are unchanged. For the unchanged conditions it gives information about the increase in cell density, aggregate size, nutrient consumption rates, pH variation, by-product production rates, etc. In other words, for the unchanged conditions the information pertains to the set of simulated variables which comprises a change in the cell density, a change in the cell aggregate size, a rate of change in the nutrient consumption, the variation in the pH value, a rate of change in the by-product production, one or more oxygen levels, one or more carbon dioxide levels, an effect of temperature in the bio reactor. The above implementation of the optimization module 1 is better understood by way of following description.

An optimization algorithm has been implemented in the system 100 of FIGS. 1 and 2 in order to optimize the output yield from the bioreactor operation. The simulated results from the mathematical model are received here which consists of cell density, aggregate size, nutrient and by-product concentration, pH value, oxygen, and carbon dioxide levels. More specifically, PSO (Particle Swarm Optimization) optimization algorithm has been implemented with an objective function to optimize the viable cell density. The variables that the algorithm makes changes in, are the operating parameters of the bioreactor plant such as the agitation rate, sparging flow rate, macro-spargers flow rate, feed rate, base addition, temperature controller. NSGA II (Nondominated Sorting Genetic Algorithm) algorithm can also be used instead of PSO algorithm. The constraints put here are on the operating limits of the operating parameters. For example, limits of agitation rate depend on the impeller and motor equipment installed, sparging flow rate lower and upper limits. The new parameter values from the optimization module 1 are sent back to the mathematical to simulate the cell growth and evaluate cell density and other constraint values. This is an iterative process which aims at maximizing the stem cell density while satisfying the constraints. The optimized operating parameters are the key operating parameter values. These values along with complete simulation data of output parameters are sent for further processing.

Referring to steps of FIG. 3, at step 212 of the method of the present disclosure, the one or more hardware processors 104 control a variation in a pH value, and a cell aggregate size using one or more control strategies based on the set of simulated variables for the growth of the one or more human stem cells. The one or more control strategies are pH value control strategy and cell aggregate size control strategy. The variation in the pH value ranges from a first pre-defined value to a second pre-defined value. pH range values depend on the set-points that are agreed upon to run a cell culture operation. This pH set-points act as a reference value for the control strategies. It is a mean value with some tolerance within which the pH variation can be tolerated by the cell culture. Beyond the tolerance, the growth of the cells is adversely affected. An example of pH setpoint is 6.80-7.00 with the mean pH level being 6.90, 6.95-7.15 with the mean pH level being 7.05. It is to be understood by a person having ordinary skill in the art or person skilled in the art that the system 100 may employ a control strategy from the one or more control strategies which can control both the variation in the pH value, and the cell aggregate size, in an embodiment of the present disclosure. Alternatively, the variation in the pH value, and the cell aggregate size may be controlled by respective control strategies. pH control module implements one of the two control strategies available which are: Sparging and base addition control; Only sparging control. The feasibility of implementing any of those strategies is provided by the pH control module. pH control module after receiving the pH variation as an input from the model library decides on the control strategy. The decisions made here are the flow rate of base addition feed, sparging rate and macro-sparging rate and their variation with time according to the pH variation predicted. Aggregate size controller is triggered whenever the averaged aggregate size is predicted to go beyond a threshold value (350-400 micrometer). When triggered, an enzyme is released into the cell culture medium which aids in cell aggregate break-up. This enzyme can be Botolinum Hemagglutinin, in one example embodiment of the present disclosure. It is to be understood by a person having ordinary skill in the art or person skilled in the art that such examples of Botolinum Hemagglutinin/enzyme used herein shall not be construed as limiting the scope of the present disclosure. The control strategy decisions are made in order to control the pH and keep it in the range which is most favorable for human stem cells growth/expansion. This control strategy decision is in the form of changing the macro-spargers flow rate, base addition into the bioreactor cell culture medium, etc.

The simulation results (e.g., the set of simulated variables) are passed onto the optimization module 1 where operating parameters are determined for maximization of the viable cell density for the given set of initial conditions (inputs to the mathematic model). Here the operating parameters are changed which are then given to the control module for its execution. The mathematical model parameters are defined as a function of temperature through which the effect of temperature is captured. Thus, the bioreactors installed at various geographical locations can be integrated with the mathematical model for human stem cells growth/expansion.

At step 214 of the method of the present disclosure, the one or more hardware processors 104 perform a comparison of the set of optimized real-time operating variables and the second set of parameters to obtain a set of correction factors. At step 216 of the method of the present disclosure, the one or more hardware processors 104 send a plurality of recommended parameter values to a plant automation system based on the set of correction factors. At step 218 of the method of the present disclosure, the one or more hardware processors 104 update the second set of parameters based on the plurality of recommended parameter values. The above steps of 214 through 218 are better understood by way of following description:

In case of positive feasibility outcome, the set of optimized real-time operating variables and the second set of parameters are received as input for comparison. Other inputs include simulated information on expansion of human stem cells and optimized key parameter values. If there is no deviation observed between the set of optimized real-time operating variables and the second set of parameters, the same control strategy is employed and this is conveyed to a global control unit (e.g., refer FIG. 2). Further, same is sent to the pH control module and aggregate disruptor module where no change in the strategy takes place. In case there is deviation in the set of optimized real-time operating variables and the second set of parameters based on the comparison, it indicates that the bioreactor operation parameters need some corrections. This deviation can be of many reasons which may not be in control. For deviation cases, the amount of deviation is sent to the global control unit. At the same time, the global control unit receives the bioreactor operating parameter values from a monitoring unit (e.g., refer FIG. 2). For the deviation observed, corrective measures are then by the global control unit. The global control unit further comprises various control units (e.g., refer C1, C2, C3, C4, and the like), which control temperature, agitation (or agitation rate), feed rate, sparger, and the like, respectively. For instance, C1 controls temperature of the bioreactor, C2 controls agitation, C3 controls feed rate, and C4 controls sparger. It is to be understood by a person having ordinary skill in the art or person skilled in the art that number of control units as mentioned above are configurable and may vary depending upon the requirement and set up of the system 100 for a given environment. The global control unit, the pH control module, and an aggregate disruptive module are better understood by way of following description:

The global control unit takes in the input such as the set of correction factors sends the plurality of recommended parameter values (e.g., recommended operating parameter) to the plant automation system. It sends instructions and these are dynamic as it keeps on changing as the real-time data is fed onto the system 100. The global control unit takes corrective actions by changing the control strategy. For example, the oxygen concentration from the soft sensor data comes out to be significantly lesser than the simulated data value for the same time stamp, the sparging is increased into the system so that oxygen level is restored, and the growth trajectory is corrected to achieve the target cell density value. For cases where there is no significant deviation, the control strategy remains unchanged until a deviation is observed.

The pH control module implements 2 pH control strategies: (i) sparging and base-addition controls, and (ii) only sparging control where macro-spargers are used. pH prediction is dependent on the physics of cell metabolism which is different for mammalian cells and human stem cells. This difference is taken care of by the mathematical model. Feasibility of implementing the above two pH control strategies is provided by the pH control module. The pH control module takes the prediction of pH level from the mathematic model as an input. Depending on the variation of the pH level, it decides on the control strategy and accordingly works in tandem with the optimization module for that given strategy to come up with optimized key parameters. Final output from the pH control module is the control strategy and is given to the optimization module 1. The output consists of bas addition feed rate, sparging rate or macro-sparging flow rate. The optimization module 1 optimizes for the cell density and iteratively works with the model to converge on a final set of optimized variables (e.g., the set of optimized real-time operating variables).

The aggregate disruption module (or the one or more hardware processors 104) takes the predicted cell aggregate size, cell density and target cell density from the mathematical model as an input. The aggregate disruption module gets triggered when the average aggregate size of cell aggregate grows beyond a threshold value (e.g., say 350 - 400 micrometer). This decision is affected by the current cell aggregate size, cell density and target cell density from the whole culture. The aggregate disruption module considers all these factors and accordingly sends the recommended parameter values (or recommendations) to the plant automation system. When the aggregate disruption module is triggered, Botulinum hemagglutinin is added for aggregate break-up. This feed instruction is given to the global control unit which is then carried out by the plant automation system. The plant automation system performs the last step which is making changes in the bioreactor operating parameters. The plant automation system receives recommendations and performs the required action like changing the sparging rate, feed rate, etc.

It is to be understood by a person having ordinary skill in the art or person skilled in the art that user alert for initial conditions is triggered when the initial conditions are not suitable for the given target cell density that is required to achieve. In this case, the mathematical model with optimization module 2, itself suggests some changes to be made in the initial conditions to achieve the target output. The alert is to be acted upon by the bioreactor system operator and necessary adjustments must be made before starting the cell culture process.

Bioreactors are vessels or closed containers where the cells are cultured. These cells can be mammalian cells to produce proteins, mAb etc or they may be microbial cells, biomass, etc. These bioreactors can also be used for growth/expansion of human stem cells. Bioreactors are of different types such as horizontal stirred vessel, spinner flasks for clinical laboratory purposes, vertical wheel bioreactors. Main differences between the bioreactors arise in hydrodynamics of the cell culture media, impeller geometry, impeller speed, mixing efficiency, shear rate, turbulence etc. These differences are taken into consideration by the mathematical model of the system and method of the present disclosure. Impeller geometry and its speed are taken as a constraint in the optimization module (1 and 2) according to the bioreactor type in use. Significant effect has been observed for mixing efficiency and shear rate as hydrodynamics differ for different bioreactor types. Horizontal stirred vessel bioreactors have much lesser mixing efficiency than Vertical wheel bioreactors (VWBR). This is reflected on the kLa value (e.g., volumetric mass transfer coefficient (kLa) is a parameter that determines the rate at which a gaseous compound (e.g., O2 or CO2) can transfer between the gas phase and the liquid phase) which is calculated for capturing oxygen transfer rate and is used in the mathematical model. Much more uniform hydrodynamics nature is observed in VWBR than in horizontal stirred wheel bioreactor due to lemniscate mixing pattern observed in VWBR. Thus, the cells cultured in horizontal wheel bioreactor experience more sharper gradients across the cell culture media. The mathematical model handles this situation by taking inputs from critical regions of the bioreactor, pre-processing the inputs and coming up with an optimized key operating values for obtaining maximum possible yield of human stem cells. The mathematical model is implemented in such a way that it avoids extreme growth cases in the bioreactor while ensuring uniformity and better quality.

### EXPERIMENTAL RESULTS:

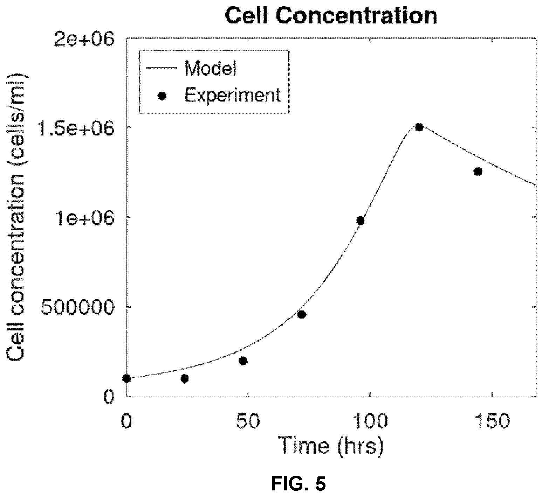

The physics-based mathematical model has been validated with the experimental data available in literature (e.g., Bartolini's literature - refer https://www.sciencedirect.com/science/article/abs/pii/50263876215002592; DOI: https://doi.org/10.1016/j.cherd.2015.07.014). This data is for batch operation of bioreactor. The mathematic model predicted well as is observed from FIG. 5 for the batch operation of the bioreactor. More specifically, FIG. 5, with reference to FIGS. 1 through 4, depicts a graphical representation illustrating a cell concentration profile with respect to time for the bioreactor, in accordance with an embodiment of the present disclosure.

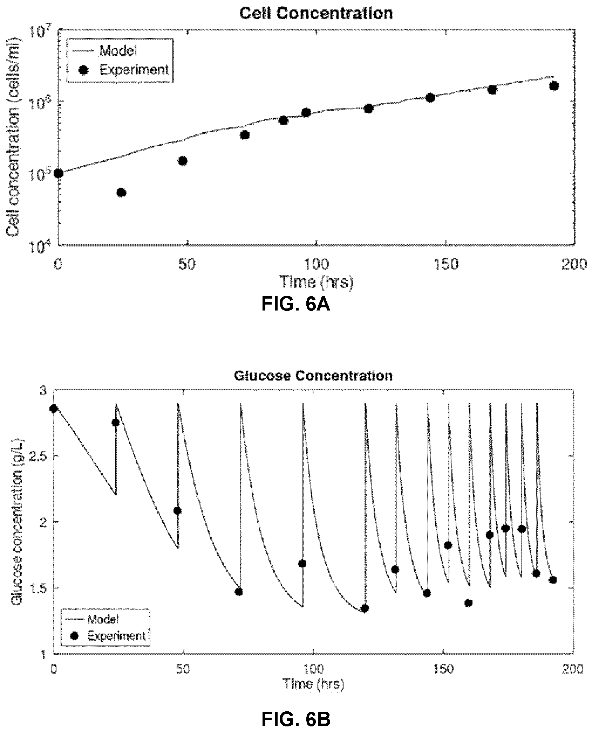

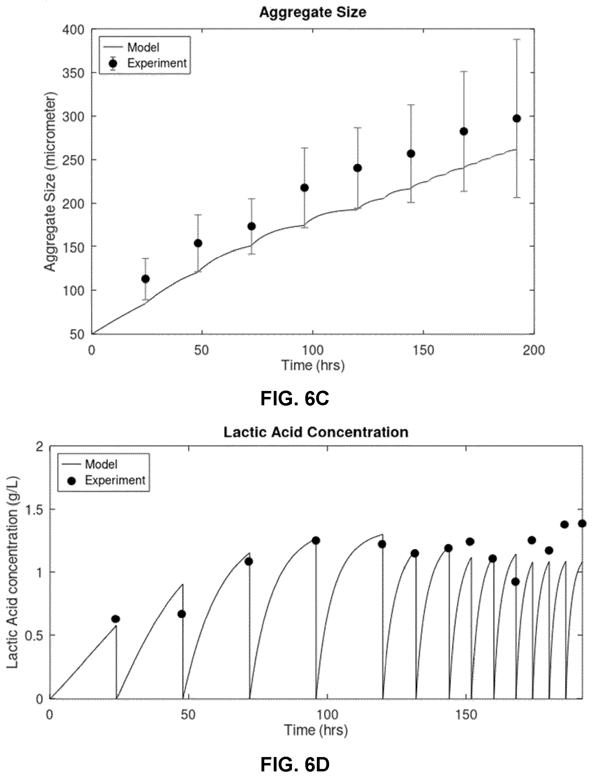

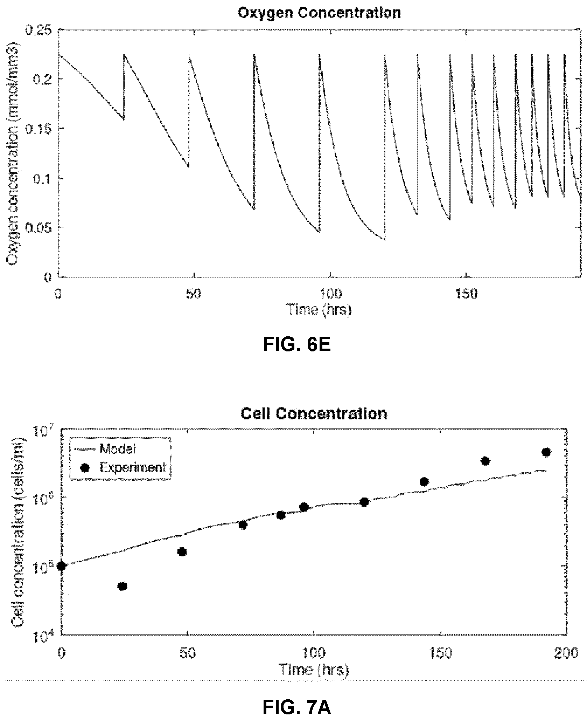

FIGS. 6A through 6E, with reference to FIGS. 1 through 5, depict a graphical representation illustrating various profiles for a non-aggregate disruption (NAD) scenario of the bioreactor, in accordance with an embodiment of the present disclosure. More specifically, FIG. 6A depicts a graphical representation illustrating a cell concentration profile for a non-aggregate disruption (NAD) scenario of the bioreactor, in accordance with an embodiment of the present disclosure. FIG. 6B depicts a graphical representation illustrating a glucose concentration profile for the non-aggregate disruption (NAD) scenario of the bioreactor, in accordance with an embodiment of the present disclosure. FIG. 6C depicts a graphical representation illustrating an aggregate cell size for the non-aggregate disruption (NAD) scenario of the bioreactor, in accordance with an embodiment of the present disclosure. FIG. 6D depicts a graphical representation illustrating a lactic acid concentration profile for the non-aggregate disruption (NAD) scenario of the bioreactor, in accordance with an embodiment of the present disclosure. FIG. 6E depicts a graphical representation illustrating an oxygen concentration profile for the non-aggregate disruption (NAD) scenario of the bioreactor, in accordance with an embodiment of the present disclosure.

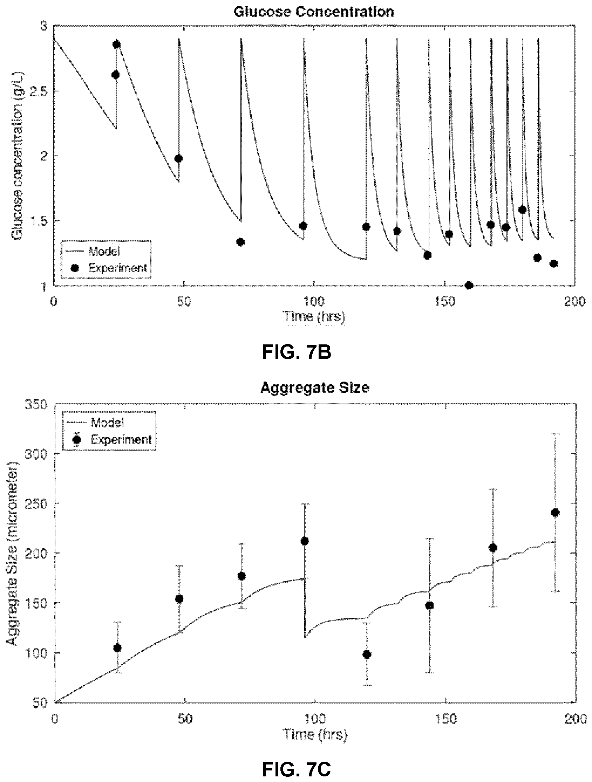

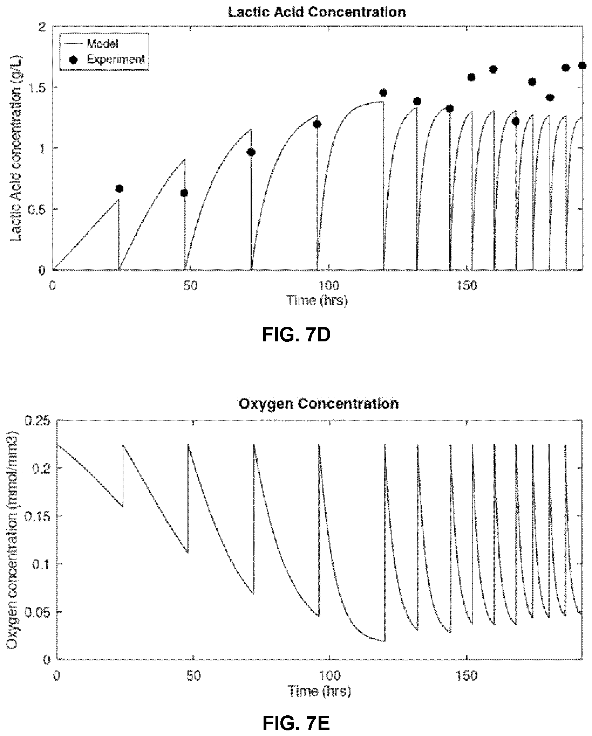

FIGS. 7A through 7E, with reference to FIGS. 1 through 6E, depict a graphical representation illustrating various profiles for an aggregate disruption (NAD) scenario of the bioreactor, in accordance with an embodiment of the present disclosure. More specifically, FIG. 7A depicts a graphical representation illustrating the cell concentration profile for the aggregate disruption (AD) scenario of the bioreactor, in accordance with an embodiment of the present disclosure. FIG. 7B depicts a graphical representation illustrating the glucose concentration profile for the aggregate disruption (AD) scenario of the bioreactor, in accordance with an embodiment of the present disclosure. FIG. 7C depicts a graphical representation illustrating the aggregate cell size for the aggregate disruption (AD) scenario of the bioreactor, in accordance with an embodiment of the present disclosure. FIG. 7D depicts a graphical representation illustrating the lactic acid concentration profile for the aggregate disruption (AD) scenario of the bioreactor, in accordance with an embodiment of the present disclosure. FIG. 7E depicts a graphical representation illustrating an oxygen concentration profile for the aggregate disruption (AD) scenario of the bioreactor, in accordance with an embodiment of the present disclosure.

The physics-based mathematical model has been validated with the experimental data from Galvanauskas literature as known in the art (e.g., refer https://www.sciencedirect.com/science/article/pii/S2352320418301068; DOI: https://doi.org/10.1016/j.reth.2019.04.007). The experimental data is for the fed-batch operation of the bioreactor for the culturing of hiPSCs (human induced pluripotent stem cells). The parameters compared here are cell concentration, cell aggregate size, glucose concentration, lactic acid concentration with respective experimental data. Oxygen concentration variation has been also plotted. The mathematical model can predict the growth of human stem cells with close validation with the experimental data. The results have been shown for two cases one with aggregate disruption and one without aggregate disruption. It is observed that the mathematical model predicts well for both the situations and that cell growth is enhanced for aggregate disruption case. With the validation results, the mathematical model predicts well for both batch as well as fed batch conditions.

Human stem cells are more sensitive to the cell culture environment as compared to other cell types. Although they have a great pluripotent potential, a slight change in the culture environment can give rise to uncontrolled differentiation of the cells which degrades the quality of the end product. With these challenges large-scale expansion of human stem cells has been limited which the present disclosure and its system and method address. The system and method optimize the bioreactor by performing various simulation, and optimization to uniform hydrodynamics environment inside the bioreactor. This ensures uniform distribution of cells, nutrients which results in uniformity in the product yield and helps in easy predictability of the quality.

Further, with limited knowledge on the phenomenon of stem cell growth and differentiation, the system and method of the present disclosure provide the next step forward in understanding the underlying physics of human stem cell expansion. The mathematical model implemented by the system and method captures multiple physics and works with bioreactor plant data to optimize the operating condition which favors human stem cell expansion/growth. This is particularly significant for such sensitive and complex cell expansion process. The mathematical model is validated through experimental results (shown through graphs/figures and description above) and further proves that it is able to capture the physics and with integration with the bioreactor plant system it provides a platform for human stem cell expansion/growth. The mathematical model as implemented by the system and method of the present disclosure is capable of handling different bioreactor operations such as batch, fed-batch, continuous fed-batch, etc., as mentioned above.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method comprising:
receiving, via one or more hardware processors of a bioreactor, a cell culture medium of one or more human stem cells and a sparger supply (202);
obtaining, via the one or more hardware processors, by using a plurality of soft sensors placed at one or more regions of the bioreactor, a soft sensor data pertaining to a first set of parameters and a second set of parameters from the cell culture medium, wherein the first set of parameters comprises an inoculum density, a cell aggregate size, a pH value, a nutrient concentration, a temperature, a by-product concentration, an agitation rate, a sparging rate of the sparger supply, a macro-sparger flow rate, and a feed rate, wherein the second set of parameters comprises a base-addition controller, and wherein the second set of parameters further comprises at least a subset of the first set of parameters (204);
pre-processing, via the one or more hardware processors, the soft sensor data to obtain a plurality of pre-processed sensor values (206);
performing simulation, via the one or more hardware processors, by using a physics based mathematical model, on the plurality of pre-processed sensor values pertaining to the first set of parameters, and a third set of parameters to obtain a set of simulated variables, wherein the third set of parameters are obtained from a domain knowledge database (208);
iteratively optimizing, via the one or more hardware processors, the second set of parameters using the set of simulated variables to obtain a set of optimized real-time operating variables, until a value of a cell density reaches a corresponding predefined threshold, wherein the set of optimized real-time operating variables are obtained to evaluate the cell density using one or more associated constraint values (210);
controlling, via the one or more hardware processors, a variation in a pH value, and a cell aggregate size using one or more control strategies based on the set of simulated variables for the growth of the one or more human stem cells (212);
performing, via the one or more hardware processors, a comparison of the set of optimized real-time operating variables and the second set of parameters to obtain a set of correction factors (214);
sending, via the one or more hardware processors, a plurality of recommended parameter values to a plant automation system based on the set of correction factors (216); and
updating, via the one or more hardware processors, the second set of parameters based on the plurality of recommended parameter values (218).

2. The processor implemented method as claimed in claim 1, wherein the bioreactor is configured to inspect one or more key parameters pertaining to growth of the one or more human stem cells, and wherein the one or more key parameters comprises the cell density, the cell aggregate size, a nutrient concentration, a by-product concentration, and a dead cell density.

3. The processor implemented method as claimed in claim 1, wherein the step of pre-processing comprises averaging the soft sensor data obtained from the plurality of soft sensors, and wherein the soft sensor data is pre-processed to refrain bias in growth of the one or more human stem cells.

4. The processor implemented method as claimed in claim 1, wherein the variation in the pH value ranges from a first pre-defined value to a second pre-defined value.

5. The processor implemented method as claimed in claim 1, wherein the set of simulated variables comprises a change in the cell density, a change in the cell aggregate size, a rate of change in the nutrient consumption, the variation in the pH value, a rate of change in the by-product production, one or more oxygen levels, one or more carbon dioxide levels, an effect of temperature in the bio reactor.

6. A system (100), comprising:
a memory (102) storing instructions;
one or more communication interfaces (106); and
one or more hardware processors (104) coupled to the memory (102) via the one or more communication interfaces (106), wherein the one or more hardware processors (104) are configured by the instructions to:
receive a cell culture medium of one or more human stem cells and a sparger supply pertaining to a bioreactor;
obtain, by using a plurality of soft sensors placed at one or more regions of the bioreactor, a soft sensor data pertaining to a first set of parameters and a second set of parameters from the cell culture medium, wherein the first set of parameters comprises an inoculum density, a cell aggregate size, a pH value, a nutrient concentration, a temperature, a by-product concentration, an agitation rate, a sparging rate of the sparger supply, a macro-sparger flow rate, and a feed rate, wherein the second set of parameters comprises a base-addition controller, and wherein the second set of parameters further comprises at least a subset of the first set of parameters;
pre-process the soft sensor data to obtain a plurality of pre-processed sensor values;
perform simulation, by using a physics based mathematical model, on the plurality of pre-processed sensor values pertaining to the first set of parameters, and a third set of parameters to obtain a set of simulated variables, wherein the third set of parameters are obtained from a domain knowledge database;
iteratively optimize the second set of parameters using the set of simulated variables to obtain a set of optimized real-time operating variables, until a value of a cell density reaches a corresponding predefined threshold, wherein the set of optimized real-time operating variables are obtained to evaluate the cell density using one or more associated constraint values;
control, by using one or more control strategies, a variation in a pH value, and a cell aggregate size based on the set of simulated variables for the growth of the one or more human stem cells;
perform a comparison of the set of optimized real-time operating variables and the second set of parameters to obtain a set of correction factors;
send a plurality of recommended parameter values to a plant automation system based on the set of correction factors; and
update the second set of parameters based on the plurality of recommended parameter values.

7. The system as claimed in claim 6, wherein the bioreactor is configured to inspect one or more key parameters pertaining to growth of the one or more human stem cells, and wherein the one or more key parameters comprises the cell density, the cell aggregate size, a nutrient concentration, a by-product concentration, and a dead cell density.

8. The system as claimed in claim 6, wherein the step of pre-processing comprises averaging the soft sensor data obtained from the plurality of soft sensors, and wherein the soft sensor data is pre-processed to refrain bias in growth of the one or more human stem cells.

9. The system as claimed in claim 6, wherein the variation in the pH value ranges from a first pre-defined value to a second pre-defined value.

10. The system of claim 6, wherein the set of simulated variables comprises a change in the cell density, a change in the cell aggregate size, a rate of change in the nutrient consumption, the variation in the pH value, a rate of change in the by-product production, one or more oxygen levels, one or more carbon dioxide levels, an effect of temperature in the bio reactor.

11. One or more non-transitory machine-readable information storage mediums comprising one or more instructions which when executed by one or more hardware processors cause:
receiving, by a bioreactor, a cell culture medium of one or more human stem cells and a sparger supply;
obtaining, by using a plurality of soft sensors, placed at one or more regions of the bioreactor, a soft sensor data pertaining to a first set of parameters and a second set of parameters from the cell culture medium, wherein the first set of parameters comprises an inoculum density, a cell aggregate size, a pH value, a nutrient concentration, a temperature, a by-product concentration, an agitation rate, a sparging rate of the sparger supply, a macro-sparger flow rate, and a feed rate, wherein the second set of parameters comprises a base-addition controller, and wherein the second set of parameters further comprises at least a subset of the first set of parameters;
pre-processing the soft sensor data to obtain a plurality of pre-processed sensor values;
performing simulation, by using a physics based mathematical model, on the plurality of pre-processed sensor values pertaining to the first set of parameters, and a third set of parameters to obtain a set of simulated variables, wherein the third set of parameters are obtained from a domain knowledge database;
iteratively optimizing the second set of parameters using the set of simulated variables to obtain a set of optimized real-time operating variables, until a value of a cell density reaches a corresponding predefined threshold, wherein the set of optimized real-time operating variables are obtained to evaluate the cell density using one or more associated constraint values;
controlling a variation in a pH value, and a cell aggregate size using one or more control strategies based on the set of simulated variables for the growth of the one or more human stem cells;
performing a comparison of the set of optimized real-time operating variables and the second set of parameters to obtain a set of correction factors;
sending a plurality of recommended parameter values to a plant automation system based on the set of correction factors; and
updating the second set of parameters based on the plurality of recommended parameter values.

12. The one or more non-transitory machine-readable information storage mediums as claimed in claim 11, wherein the bioreactor is configured to inspect one or more key parameters pertaining to growth of the one or more human stem cells, and wherein the one or more key parameters comprises the cell density, the cell aggregate size, a nutrient concentration, a by-product concentration, and a dead cell density.

13. The one or more non-transitory machine-readable information storage mediums as claimed in claim 11, wherein the step of pre-processing comprises averaging the soft sensor data obtained from the plurality of soft sensors, and wherein the soft sensor data is pre-processed to refrain bias in growth of the one or more human stem cells.

14. The one or more non-transitory machine-readable information storage mediums as claimed in claim 11, wherein the variation in the pH value ranges from a first pre-defined value to a second pre-defined value.

15. The one or more non-transitory machine-readable information storage mediums as claimed in claim 11, wherein the set of simulated variables comprises a change in the cell density, a change in the cell aggregate size, a rate of change in the nutrient consumption, the variation in the pH value, a rate of change in the by-product production, one or more oxygen levels, one or more carbon dioxide levels, an effect of temperature in the bio reactor.

## Patentansprüche

1. Prozessorimplementiertes Verfahren, umfassend:
Empfangen, über einen oder mehrere Hardwareprozessoren eines Bioreaktors, eines Zellkulturmediums von einer oder mehreren menschlichen Stammzellen und eines Spargervorrats (202);
Erhalten, über den einen oder die mehreren Hardwareprozessoren, unter Verwendung einer Mehrzahl von Weichsensoren, die an einer oder mehreren Regionen des Bioreaktors platziert sind, von Weichsensordaten, die sich auf einen ersten Satz von Parametern und einen zweiten Satz von Parametern beziehen, aus dem Zellkulturmedium, wobei der erste Satz von Parametern eine Inokulumdichte, eine Zellaggregatgröße, einen pH-Wert, eine Nährstoffkonzentration, eine Temperatur, eine Nebenproduktkonzentration, eine Rührgeschwindigkeit, eine Begasungsgeschwindigkeit des Spargervorrats, eine Makro-Sparger-Strömungsgeschwindigkeit und eine Zufuhrgeschwindigkeit umfasst, wobei der zweite Satz von Parametern eine Basenzugabesteuerung umfasst und wobei der zweite Satz von Parametern ferner mindestens eine Teilmenge des ersten Satzes von Parametern (204) umfasst;
Vorverarbeiten, über den einen oder die mehreren Hardwareprozessoren, der Weichsensordaten, um eine Mehrzahl von vorverarbeiteten Sensorwerten (206) zu erhalten;
Durchführen einer Simulation, über den einen oder die mehreren Hardwareprozessoren, unter Verwendung eines physikbasierten mathematischen Modells, an der Mehrzahl von vorverarbeiteten Sensorwerten, die sich auf den ersten Satz von Parametern beziehen, und eines dritten Satzes von Parametern, um einen Satz von simulierten Variablen zu erhalten, wobei der dritte Satz von Parametern aus einer Domänenwissensdatenbank (208) erhalten wird;
iteratives Optimieren, über den einen oder die mehreren Hardwareprozessoren, des zweiten Satzes von Parametern unter Verwendung des Satzes von simulierten Variablen, um einen Satz von optimierten Echtzeit-Betriebsvariablen zu erhalten, bis ein Wert einer Zelldichte einen entsprechenden vordefinierten Schwellenwert erreicht, wobei der Satz von optimierten Echtzeit-Betriebsvariablen erhalten wird, um die Zelldichte unter Verwendung eines oder mehrerer assoziierter Randbedingungswerte (210) zu bewerten;
Steuern, über den einen oder die mehreren Hardwareprozessoren, einer Variation in einem pH-Wert und einer Zellaggregatgröße unter Verwendung einer oder mehrerer Steuerstrategien basierend auf dem Satz von simulierten Variablen für das Wachstum der einen oder mehreren menschlichen Stammzellen (212);
Durchführen, über den einen oder die mehreren Hardwareprozessoren, eines Vergleichs des Satzes von optimierten Echtzeit-Betriebsvariablen und des zweiten Satzes von Parametern, um einen Satz von Korrekturfaktoren (214) zu erhalten;
Senden, über den einen oder die mehreren Hardwareprozessoren, einer Mehrzahl von empfohlenen Parameterwerten an ein Anlagenautomatisierungssystem basierend auf dem Satz von Korrekturfaktoren (216); und
Aktualisieren, über den einen oder die mehreren Hardwareprozessoren, des zweiten Satzes von Parametern basierend auf der Mehrzahl von empfohlenen Parameterwerten (218).

2. Prozessorimplementiertes Verfahren nach Anspruch 1, wobei der Bioreaktor konfiguriert ist, um einen oder mehrere Schlüsselparameter zu inspizieren, die sich auf das Wachstum der einen oder mehreren menschlichen Stammzellen beziehen, und wobei der eine oder die mehreren Schlüsselparameter die Zelldichte, die Zellaggregatgröße, eine Nährstoffkonzentration, eine Nebenproduktkonzentration und eine tote Zelldichte umfassen.

3. Prozessorimplementiertes Verfahren nach Anspruch 1, wobei der Schritt des Vorverarbeitens das Mitteln der Weichsensordaten umfasst, die von der Mehrzahl von Weichsensoren erhalten werden, und wobei die Weichsensordaten vorverarbeitet werden, um eine Verzerrung beim Wachstum der einen oder mehreren menschlichen Stammzellen zu unterlassen.

4. Prozessorimplementiertes Verfahren nach Anspruch 1, wobei die Variation im pH-Wert von einem ersten vordefinierten Wert bis zu einem zweiten vordefinierten Wert reicht.

5. Prozessorimplementiertes Verfahren nach Anspruch 1, wobei der Satz von simulierten Variablen eine Änderung der Zelldichte, eine Änderung der Zellaggregatgröße, eine Änderungsrate des Nährstoffverbrauchs, die Variation im pH-Wert, eine Änderungsrate der Nebenproduktproduktion, einen oder mehrere Sauerstoffwerte, einen oder mehrere Kohlendioxidwerte, einen Effekt der Temperatur im Bioreaktor umfasst.

6. System (100), umfassend:
einen Speicher (102), der Anweisungen speichert;
eine oder mehrere Kommunikationsschnittstellen (106); und
einen oder mehrere Hardwareprozessoren (104), die mit dem Speicher (102) über die eine oder die mehreren Kommunikationsschnittstellen (106) gekoppelt sind, wobei der eine oder die mehreren Hardwareprozessoren (104) durch die Anweisungen konfiguriert sind zum:
Empfangen eines Zellkulturmediums von einer oder mehreren menschlichen Stammzellen und eines Spargervorrats, der sich auf einen Bioreaktor bezieht;
Erhalten, unter Verwendung einer Mehrzahl von Weichsensoren, die an einer oder mehreren Regionen des Bioreaktors platziert sind, von Weichsensordaten, die sich auf einen ersten Satz von Parametern und einen zweiten Satz von Parametern beziehen, aus dem Zellkulturmedium, wobei der erste Satz von Parametern eine Inokulumdichte, eine Zellaggregatgröße, einen pH-Wert, eine Nährstoffkonzentration, eine Temperatur, eine Nebenproduktkonzentration, eine Rührgeschwindigkeit, eine Begasungsgeschwindigkeit des Spargervorrats, eine Makro-Sparger-Strömungsgeschwindigkeit und eine Zufuhrgeschwindigkeit umfasst, wobei der zweite Satz von Parametern eine Basenzugabesteuerung umfasst und wobei der zweite Satz von Parametern ferner mindestens eine Teilmenge des ersten Satzes von Parametern umfasst;
Vorverarbeiten der Weichsensordaten, um eine Mehrzahl von vorverarbeiteten Sensorwerten zu erhalten;
Durchführen einer Simulation, unter Verwendung eines physikbasierten mathematischen Modells, an der Mehrzahl von vorverarbeiteten Sensorwerten, die sich auf den ersten Satz von Parametern beziehen, und eines dritten Satzes von Parametern, um einen Satz von simulierten Variablen zu erhalten, wobei der dritte Satz von Parametern aus einer Domänenwissensdatenbank erhalten wird;
iteratives Optimieren des zweiten Satzes von Parametern unter Verwendung des Satzes von simulierten Variablen, um einen Satz von optimierten Echtzeit-Betriebsvariablen zu erhalten, bis ein Wert einer Zelldichte einen entsprechenden vordefinierten Schwellenwert erreicht, wobei der Satz von optimierten Echtzeit-Betriebsvariablen erhalten wird, um die Zelldichte unter Verwendung eines oder mehrerer assoziierter Randbedingungswerte zu bewerten;
Steuern, unter Verwendung einer oder mehrerer Steuerstrategien, einer Variation in einem pH-Wert und einer Zellaggregatgröße basierend auf dem Satz von simulierten Variablen für das Wachstum der einen oder mehreren menschlichen Stammzellen;
Durchführen eines Vergleichs des Satzes von optimierten Echtzeit-Betriebsvariablen und des zweiten Satzes von Parametern, um einen Satz von Korrekturfaktoren zu erhalten;
Senden einer Mehrzahl von empfohlenen Parameterwerten an ein Anlagenautomatisierungssystem basierend auf dem Satz von Korrekturfaktoren; und
Aktualisieren des zweiten Satzes von Parametern basierend auf der Mehrzahl von empfohlenen Parameterwerten.

7. System nach Anspruch 6, wobei der Bioreaktor konfiguriert ist, um einen oder mehrere Schlüsselparameter zu inspizieren, die sich auf das Wachstum der einen oder mehreren menschlichen Stammzellen beziehen, und wobei der eine oder die mehreren Schlüsselparameter die Zelldichte, die Zellaggregatgröße, eine Nährstoffkonzentration, eine Nebenproduktkonzentration und eine tote Zelldichte umfassen.

8. System nach Anspruch 6, wobei der Schritt des Vorverarbeitens das Mitteln der Weichsensordaten umfasst, die von der Mehrzahl von Weichsensoren erhalten werden, und wobei die Weichsensordaten vorverarbeitet werden, um eine Verzerrung beim Wachstum der einen oder mehreren menschlichen Stammzellen zu unterlassen.

9. System nach Anspruch 6, wobei die Variation im pH-Wert von einem ersten vordefinierten Wert bis zu einem zweiten vordefinierten Wert reicht.

10. System nach Anspruch 6, wobei der Satz von simulierten Variablen eine Änderung der Zelldichte, eine Änderung der Zellaggregatgröße, eine Änderungsrate des Nährstoffverbrauchs, die Variation im pH-Wert, eine Änderungsrate der Nebenproduktproduktion, einen oder mehrere Sauerstoffwerte, einen oder mehrere Kohlendioxidwerte, einen Effekt der Temperatur im Bioreaktor umfasst.

11. Ein oder mehrere nichtflüchtige maschinenlesbare Informationsspeichermedien, die eine oder mehrere Anweisungen umfassen, die, wenn sie von einem oder mehreren Hardwareprozessoren ausgeführt werden, bewirken:
Empfangen, durch einen Bioreaktor, eines Zellkulturmediums von einer oder mehreren menschlichen Stammzellen und eines Spargervorrats;
Erhalten, unter Verwendung einer Mehrzahl von Weichsensoren, die an einer oder mehreren Regionen des Bioreaktors platziert sind, von Weichsensordaten, die sich auf einen ersten Satz von Parametern und einen zweiten Satz von Parametern beziehen, aus dem Zellkulturmedium, wobei der erste Satz von Parametern eine Inokulumdichte, eine Zellaggregatgröße, einen pH-Wert, eine Nährstoffkonzentration, eine Temperatur, eine Nebenproduktkonzentration, eine Rührgeschwindigkeit, eine Begasungsgeschwindigkeit des Spargervorrats, eine Makro-Sparger-Strömungsgeschwindigkeit und eine Zufuhrgeschwindigkeit umfasst, wobei der zweite Satz von Parametern eine Basenzugabesteuerung umfasst und wobei der zweite Satz von Parametern ferner mindestens eine Teilmenge des ersten Satzes von Parametern umfasst;
Vorverarbeiten der Weichsensordaten, um eine Mehrzahl von vorverarbeiteten Sensorwerten zu erhalten;
Durchführen einer Simulation, unter Verwendung eines physikbasierten mathematischen Modells, an der Mehrzahl von vorverarbeiteten Sensorwerten, die sich auf den ersten Satz von Parametern beziehen, und eines dritten Satzes von Parametern, um einen Satz von simulierten Variablen zu erhalten, wobei der dritte Satz von Parametern aus einer Domänenwissensdatenbank erhalten wird;
iteratives Optimieren des zweiten Satzes von Parametern unter Verwendung des Satzes von simulierten Variablen, um einen Satz von optimierten Echtzeit-Betriebsvariablen zu erhalten, bis ein Wert einer Zelldichte einen entsprechenden vordefinierten Schwellenwert erreicht, wobei der Satz von optimierten Echtzeit-Betriebsvariablen erhalten wird, um die Zelldichte unter Verwendung eines oder mehrerer assoziierter Randbedingungswerte zu bewerten;
Steuern einer Variation in einem pH-Wert und einer Zellaggregatgröße unter Verwendung einer oder mehrerer Steuerstrategien basierend auf dem Satz von simulierten Variablen für das Wachstum der einen oder mehreren menschlichen Stammzellen;
Durchführen eines Vergleichs des Satzes von optimierten Echtzeit-Betriebsvariablen und des zweiten Satzes von Parametern, um einen Satz von Korrekturfaktoren zu erhalten;
Senden einer Mehrzahl von empfohlenen Parameterwerten an ein Anlagenautomatisierungssystem basierend auf dem Satz von Korrekturfaktoren; und
Aktualisieren des zweiten Satzes von Parametern basierend auf der Mehrzahl von empfohlenen Parameterwerten.

12. Ein oder mehrere nichtflüchtige maschinenlesbare Informationsspeichermedien nach Anspruch 11, wobei der Bioreaktor konfiguriert ist, um einen oder mehrere Schlüsselparameter zu inspizieren, die sich auf das Wachstum der einen oder mehreren menschlichen Stammzellen beziehen, und wobei der eine oder die mehreren Schlüsselparameter die Zelldichte, die Zellaggregatgröße, eine Nährstoffkonzentration, eine Nebenproduktkonzentration und eine tote Zelldichte umfassen.

13. Ein oder mehrere nichtflüchtige maschinenlesbare Informationsspeichermedien nach Anspruch 11, wobei der Schritt des Vorverarbeitens das Mitteln der Weichsensordaten umfasst, die von der Mehrzahl von Weichsensoren erhalten werden, und wobei die Weichsensordaten vorverarbeitet werden, um eine Verzerrung beim Wachstum der einen oder mehreren menschlichen Stammzellen zu unterlassen.

14. Ein oder mehrere nichtflüchtige maschinenlesbare Informationsspeichermedien nach Anspruch 11, wobei die Variation im pH-Wert von einem ersten vordefinierten Wert bis zu einem zweiten vordefinierten Wert reicht.

15. Ein oder mehrere nichtflüchtige maschinenlesbare Informationsspeichermedien nach Anspruch 11, wobei der Satz von simulierten Variablen eine Änderung der Zelldichte, eine Änderung der Zellaggregatgröße, eine Änderungsrate des Nährstoffverbrauchs, die Variation im pH-Wert, eine Änderungsrate der Nebenproduktproduktion, einen oder mehrere Sauerstoffwerte, einen oder mehrere Kohlendioxidwerte, einen Effekt der Temperatur im Bioreaktor umfasst.

## Revendications

1. Procédé mis en oeuvre par processeur comprenant :
la réception, par l'intermédiaire d'un ou de plusieurs processeurs matériels d'un bioréacteur, d'un milieu de culture cellulaire d'une ou de plusieurs cellules souches humaines et d'une alimentation de sparger (202) ;
l'obtention, par l'intermédiaire des un ou plusieurs processeurs matériels, en utilisant une pluralité de capteurs souples placés au niveau d'une ou de plusieurs régions du bioréacteur, de données de capteur souple se rapportant à un premier ensemble de paramètres et à un deuxième ensemble de paramètres à partir du milieu de culture cellulaire, dans lequel le premier ensemble de paramètres comprend une densité d'inoculum, une taille d'agrégat cellulaire, une valeur de pH, une concentration en nutriments, une température, une concentration en sous-produits, un taux d'agitation, un taux de barbotage de l'alimentation de sparger, un débit de macro-sparger, et un débit d'alimentation, dans lequel le deuxième ensemble de paramètres comprend un contrôleur d'addition de base, et dans lequel le deuxième ensemble de paramètres comprend en outre au moins un sous-ensemble du premier ensemble de paramètres (204) ;
le prétraitement, par l'intermédiaire des un ou plusieurs processeurs matériels, des données de capteur souple pour obtenir une pluralité de valeurs de capteur prétraitées (206) ;
la réalisation d'une simulation, par l'intermédiaire des un ou plusieurs processeurs matériels, en utilisant un modèle mathématique basé sur la physique, sur la pluralité de valeurs de capteur prétraitées se rapportant au premier ensemble de paramètres, et à un troisième ensemble de paramètres pour obtenir un ensemble de variables simulées, dans lequel le troisième ensemble de paramètres est obtenu à partir d'une base de données de connaissances de domaine (208) ;
l'optimisation itérative, par l'intermédiaire des un ou plusieurs processeurs matériels, du deuxième ensemble de paramètres en utilisant l'ensemble de variables simulées pour obtenir un ensemble de variables de fonctionnement en temps réel optimisées, jusqu'à ce qu'une valeur d'une densité cellulaire atteigne un seuil prédéfini correspondant, dans lequel l'ensemble de variables de fonctionnement en temps réel optimisées est obtenu pour évaluer la densité cellulaire en utilisant une ou plusieurs valeurs de contrainte associées (210) ;
la commande, par l'intermédiaire des un ou plusieurs processeurs matériels, d'une variation d'une valeur de pH, et d'une taille d'agrégat cellulaire en utilisant une ou plusieurs stratégies de commande basées sur l'ensemble de variables simulées pour la croissance des une ou plusieurs cellules souches humaines (212) ;
la réalisation, par l'intermédiaire des un ou plusieurs processeurs matériels, d'une comparaison de l'ensemble de variables de fonctionnement en temps réel optimisées et du deuxième ensemble de paramètres pour obtenir un ensemble de facteurs de correction (214) ;
l'envoi, par l'intermédiaire des un ou plusieurs processeurs matériels, d'une pluralité de valeurs de paramètre recommandées à un système d'automatisation d'usine sur la base de l'ensemble de facteurs de correction (216) ; et
la mise à jour, par l'intermédiaire des un ou plusieurs processeurs matériels, du deuxième ensemble de paramètres sur la base de la pluralité de valeurs de paramètre recommandées (218).

2. Procédé mis en oeuvre par processeur selon la revendication 1, dans lequel le bioréacteur est configuré pour inspecter un ou plusieurs paramètres clés concernant la croissance des une ou plusieurs cellules souches humaines, et dans lequel les un ou plusieurs paramètres clés comprennent la densité cellulaire, la taille d'agrégat cellulaire, une concentration en nutriments, une concentration en sous-produits, et une densité de cellules mortes.

3. Procédé mis en oeuvre par processeur selon la revendication 1, dans lequel l'étape de prétraitement comprend le calcul de la moyenne des données de capteur souple obtenues à partir de la pluralité de capteurs souples, et dans lequel les données de capteur souple sont prétraitées pour s'abstenir de biais dans la croissance des une ou plusieurs cellules souches humaines.

4. Procédé mis en oeuvre par processeur selon la revendication 1, dans lequel la variation de la valeur de pH va d'une première valeur prédéfinie à une deuxième valeur prédéfinie.

5. Procédé mis en oeuvre par processeur selon la revendication 1, dans lequel l'ensemble de variables simulées comprend un changement de la densité cellulaire, un changement de la taille d'agrégat cellulaire, un taux de changement de la consommation de nutriments, la variation de la valeur de pH, un taux de changement de la production de sous-produits, un ou plusieurs niveaux d'oxygène, un ou plusieurs niveaux de dioxyde de carbone, un effet de température dans le bioréacteur.

6. Système (100), comprenant :
une mémoire (102) stockant des instructions ;
une ou plusieurs interfaces de communication (106) ; et
un ou plusieurs processeurs matériels (104) couplés à la mémoire (102) par l'intermédiaire des une ou plusieurs interfaces de communication (106), dans lequel les un ou plusieurs processeurs matériels (104) sont configurés par les instructions pour :
recevoir un milieu de culture cellulaire d'une ou de plusieurs cellules souches humaines et une alimentation de sparger concernant un bioréacteur ;
obtenir, en utilisant une pluralité de capteurs souples placés au niveau d'une ou de plusieurs régions du bioréacteur, des données de capteur souple se rapportant à un premier ensemble de paramètres et à un deuxième ensemble de paramètres à partir du milieu de culture cellulaire, dans lequel le premier ensemble de paramètres comprend une densité d'inoculum, une taille d'agrégat cellulaire, une valeur de pH, une concentration en nutriments, une température, une concentration en sous-produits, un taux d'agitation, un taux de barbotage de l'alimentation de sparger, un débit de macro-sparger, et un débit d'alimentation, dans lequel le deuxième ensemble de paramètres comprend un contrôleur d'addition de base, et dans lequel le deuxième ensemble de paramètres comprend en outre au moins un sous-ensemble du premier ensemble de paramètres ;
prétraiter les données de capteur souple pour obtenir une pluralité de valeurs de capteur prétraitées ;
réaliser une simulation, en utilisant un modèle mathématique basé sur la physique, sur la pluralité de valeurs de capteur prétraitées se rapportant au premier ensemble de paramètres, et à un troisième ensemble de paramètres pour obtenir un ensemble de variables simulées, dans lequel le troisième ensemble de paramètres est obtenu à partir d'une base de données de connaissances de domaine ;
optimiser itérativement le deuxième ensemble de paramètres en utilisant l'ensemble de variables simulées pour obtenir un ensemble de variables de fonctionnement en temps réel optimisées, jusqu'à ce qu'une valeur d'une densité cellulaire atteigne un seuil prédéfini correspondant, dans lequel l'ensemble de variables de fonctionnement en temps réel optimisées est obtenu pour évaluer la densité cellulaire en utilisant une ou plusieurs valeurs de contrainte associées ;
commander, en utilisant une ou plusieurs stratégies de commande, une variation d'une valeur de pH, et une taille d'agrégat cellulaire sur la base de l'ensemble de variables simulées pour la croissance des une ou plusieurs cellules souches humaines ;
réaliser une comparaison de l'ensemble de variables de fonctionnement en temps réel optimisées et du deuxième ensemble de paramètres pour obtenir un ensemble de facteurs de correction ;
envoyer une pluralité de valeurs de paramètre recommandées à un système d'automatisation d'usine sur la base de l'ensemble de facteurs de correction ; et
mettre à jour le deuxième ensemble de paramètres sur la base de la pluralité de valeurs de paramètre recommandées.

7. Système selon la revendication 6, dans lequel le bioréacteur est configuré pour inspecter un ou plusieurs paramètres clés concernant la croissance des une ou plusieurs cellules souches humaines, et dans lequel les un ou plusieurs paramètres clés comprennent la densité cellulaire, la taille d'agrégat cellulaire, une concentration en nutriments, une concentration en sous-produits, et une densité de cellules mortes.

8. Système selon la revendication 6, dans lequel l'étape de prétraitement comprend le calcul de la moyenne des données de capteur souple obtenues à partir de la pluralité de capteurs souples, et dans lequel les données de capteur souple sont prétraitées pour s'abstenir de biais dans la croissance des une ou plusieurs cellules souches humaines.

9. Système selon la revendication 6, dans lequel la variation de la valeur de pH va d'une première valeur prédéfinie à une deuxième valeur prédéfinie.

10. Système selon la revendication 6, dans lequel l'ensemble de variables simulées comprend un changement de la densité cellulaire, un changement de la taille d'agrégat cellulaire, un taux de changement de la consommation de nutriments, la variation de la valeur de pH, un taux de changement de la production de sous-produits, un ou plusieurs niveaux d'oxygène, un ou plusieurs niveaux de dioxyde de carbone, un effet de température dans le bioréacteur.

11. Un ou plusieurs supports de stockage d'informations lisibles par machine non transitoires comprenant une ou plusieurs instructions qui, lorsqu'elles sont exécutées par un ou plusieurs processeurs matériels, amènent :
la réception, par un bioréacteur, d'un milieu de culture cellulaire d'une ou de plusieurs cellules souches humaines et d'une alimentation de sparger ;
l'obtention, en utilisant une pluralité de capteurs souples, placés au niveau d'une ou de plusieurs régions du bioréacteur, de données de capteur souple se rapportant à un premier ensemble de paramètres et à un deuxième ensemble de paramètres à partir du milieu de culture cellulaire, dans lequel le premier ensemble de paramètres comprend une densité d'inoculum, une taille d'agrégat cellulaire, une valeur de pH, une concentration en nutriments, une température, une concentration en sous-produits, un taux d'agitation, un taux de barbotage de l'alimentation de sparger, un débit de macro-sparger, et un débit d'alimentation, dans lequel le deuxième ensemble de paramètres comprend un contrôleur d'addition de base, et dans lequel le deuxième ensemble de paramètres comprend en outre au moins un sous-ensemble du premier ensemble de paramètres ;
le prétraitement des données de capteur souple pour obtenir une pluralité de valeurs de capteur prétraitées ;
la réalisation d'une simulation, en utilisant un modèle mathématique basé sur la physique, sur la pluralité de valeurs de capteur prétraitées se rapportant au premier ensemble de paramètres, et à un troisième ensemble de paramètres pour obtenir un ensemble de variables simulées, dans lequel le troisième ensemble de paramètres est obtenu à partir d'une base de données de connaissances de domaine ;
l'optimisation itérative du deuxième ensemble de paramètres en utilisant l'ensemble de variables simulées pour obtenir un ensemble de variables de fonctionnement en temps réel optimisées, jusqu'à ce qu'une valeur d'une densité cellulaire atteigne un seuil prédéfini correspondant, dans lequel l'ensemble de variables de fonctionnement en temps réel optimisées est obtenu pour évaluer la densité cellulaire en utilisant une ou plusieurs valeurs de contrainte associées ;
la commande d'une variation d'une valeur de pH, et d'une taille d'agrégat cellulaire en utilisant une ou plusieurs stratégies de commande basées sur l'ensemble de variables simulées pour la croissance des une ou plusieurs cellules souches humaines ;
la réalisation d'une comparaison de l'ensemble de variables de fonctionnement en temps réel optimisées et du deuxième ensemble de paramètres pour obtenir un ensemble de facteurs de correction ;
l'envoi d'une pluralité de valeurs de paramètre recommandées à un système d'automatisation d'usine sur la base de l'ensemble de facteurs de correction ; et
la mise à jour du deuxième ensemble de paramètres sur la base de la pluralité de valeurs de paramètre recommandées.

12. Un ou plusieurs supports de stockage d'informations lisibles par machine non transitoires selon la revendication 11, dans lequel le bioréacteur est configuré pour inspecter un ou plusieurs paramètres clés concernant la croissance des une ou plusieurs cellules souches humaines, et dans lequel les un ou plusieurs paramètres clés comprennent la densité cellulaire, la taille d'agrégat cellulaire, une concentration en nutriments, une concentration en sous-produits, et une densité de cellules mortes.

13. Un ou plusieurs supports de stockage d'informations lisibles par machine non transitoires selon la revendication 11, dans lequel l'étape de prétraitement comprend le calcul de la moyenne des données de capteur souple obtenues à partir de la pluralité de capteurs souples, et dans lequel les données de capteur souple sont prétraitées pour s'abstenir de biais dans la croissance des une ou plusieurs cellules souches humaines.

14. Un ou plusieurs supports de stockage d'informations lisibles par machine non transitoires selon la revendication 11, dans lequel la variation de la valeur de pH va d'une première valeur prédéfinie à une deuxième valeur prédéfinie.

15. Un ou plusieurs supports de stockage d'informations lisibles par machine non transitoires selon la revendication 11, dans lequel l'ensemble de variables simulées comprend un changement de la densité cellulaire, un changement de la taille d'agrégat cellulaire, un taux de changement de la consommation de nutriments, la variation de la valeur de pH, un taux de changement de la production de sous-produits, un ou plusieurs niveaux d'oxygène, un ou plusieurs niveaux de dioxyde de carbone, un effet de température dans le bioréacteur.
